Europäisches Patentamt

European Patent Office

Office européen des brevets

(19)

(11) **EP 1 470 823 A1**

(12) **EUROPEAN PATENT APPLICATION**

(43) Date of publication:
**27.10.2004 Bulletin 2004/44**

(51) Int Cl.$^7$: **A61K 47/02**, A61K 9/00

(21) Application number: **04252228.4**

(22) Date of filing: **16.04.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **23.04.2003 US 464829 P**

(71) Applicant: **ROHM AND HAAS COMPANY**
**Philadelphia, Pennsylvania 19106-2399 (US)**

(72) Inventors:
• **Greenblatt, Gary David**
**Rydal, Pennsylvania 19046 (US)**

• **Hughes, Lyn**
**Harleysville, Pennsylvania 19438 (US)**
• **Whitman, David William**
**Harleysville, Pennsylvania 19438 (US)**

(74) Representative: **Kent, Venetia Katherine et al**
**Rohm and Haas (UK) Ltd**
**European Patent Department**
**28th. Floor, City Point**
**One Ropemaker Street**
**London EC2Y 9HS (GB)**

(54) **Polymer-clay nanocomposite for extended release of active ingredient**

(57) An extended release dosage form and an extended release polymeric dispenser containing polymer-clay nanocomposite are provided. The polymer-clay nanocomposite contains exfoliated clay platelets dispersed in a polymer matrix. The extended release dosage form contains active ingredient in the polymer-clay nanocomposite. The extended release polymeric dispenser contains a reservoir of an active ingredient partially or completely encapsulated by a polymer-clay nanocomposite layer. Also provided are a method of providing release of an active ingredient from the extended release dosage form and a method or providing release of an active ingredient from the extended release polymeric dispenser. The extended release dosage form and the extended release polymeric dispenser are useful for the application of pharmaceutical compounds, agricultural compounds, and perfumes.

## Description

The present invention relates generally to the extended release of an active ingredient. In particular, the present invention relates to a composition for the extended release of an active ingredient. The invention further relates to an extended release dosage form containing a polymer-clay nanocomposite having exfoliated clay. The invention also relates to an extended release polymeric dispenser containing a layer of the polymer-clay nanocomposite. Also provided are methods for releasing the active ingredient from the extended release dosage form and from the extended release polymeric dispenser.

In drug therapy applications, it is important to maintain the concentration of a pharmaceutically active agent in a desired range, which is set by an upper limit defined by the toxicological properties of the pharmaceutically active agent and a low limit defined by the efficacy of the pharmaceutically active agent. Further, it is desired to minimize temporal variations in the concentration of the pharmaceutically active agent during the drug therapy period in order to achieve a constant concentration close to the optimal level for treatment. Variations in the concentration can arise from the administration of the pharmaceutically active agent at periodic intervals, which leads to an increase in the level of the pharmaceutically active agent after administration, followed by a peak level, and then a decline, leading to periods of less than optimal dosing, including periods of overdosing or underdosing. Further, a patient failing to receive the pharmaceutically active agent at the prescribed intervals further exacerbates variations in the concentration.

Controlled release technologies are employed to release an active ingredient in a controlled manner, such as in response to an external stimulus or over a desired duration of time. External stimuli that can lead to changes in release rates in controlled release technologies include changes in temperature, pH, or ion concentration; or to the presence of a select chemical. Controlled release technologies, which include extended release technologies, can be employed to minimize temporal variations in the concentration of an active ingredient in a system or the environment. Extended release technologies have been employed to increase the intervals between administration of pharmaceutically active agents. Examples of controlled release technologies include diffusion based systems and dissolution based systems. In diffusion based systems, the active ingredient is contained within a matrix, such as a polymer matrix, and is released by diffusion from the matrix. The active ingredient in dissolution based systems is also contained within a matrix but is released from the matrix by dissolution or erosion of the matrix. The release of the active ingredient from a matrix can involve both diffusion and dissolution.

The article "Thermosensitive Poly(N-isopropylacrylamide)-Clay Nanocomposites with Enhanced Temperature Response", *Langmuir* Vol. 16, 9895-9899, November 15, 2000, discloses a polymer-clay nanocomposite, which contains partially intercalated and partially exfoliated clay platelets dispersed in a poly(N-isopropylacrylamide) polymer matrix. Study of the thermal phase behavior of the disclosed polymer-clay nanocomposite showed a large thermal volumetric change as well as faster transition kinetics compared to conventional poly(N-isopropylacrylamide) polymer. It was suggested in the article that disclosed polymer-clay nanocomposite is useful as controlled release devices having enhanced thermal response that provide increased release rates. The cited article does not disclose polymer-clay nanocomposites providing slow or extended release.

Although methods are known for providing extended release using polymeric matrices, desired are methods for providing release of an active ingredient for longer periods of time.

Accordingly, it is desired to provide extended release compositions and articles that provide for the release of an active ingredient for longer periods of time.

It has now been found that such an improvement is obtained by compositions and articles containing polymer-clay nanocomposites having exfoliated clay platelets dispersed within a polymer matrix.

According to the first aspect of the present invention, an extended release dosage form is provided including a polymer-clay nanocomposite having exfoliated clay platelets dispersed in a polymer matrix; and an active ingredient, which is contained within the polymer-clay nanocomposite.

A second aspect of the present invention relates to a method of releasing an active ingredient, including the steps of providing an extended release dosage form containing a polymer-clay nanocomposite having exfoliated clay platelets dispersed in a polymer matrix and the active ingredient, which is contained within the polymer-clay nanocomposite; and releasing or allowing to be released the active ingredient from the extended release dosage form.

A third aspect of the present invention provides an extended release polymeric dispenser containing a polymer-clay nanocomposite layer encapsulating a reservoir of an active ingredient; wherein the polymer-clay nanocomposite layer has exfoliated clay platelets dispersed within a polymer matrix.

A fourth aspect of the present invention relates to a method of releasing an active ingredient, including the steps of providing an extended release polymeric dispenser containing a polymer-clay nanocomposite layer encapsulating a reservoir of the active ingredient, wherein the polymer-clay nanocomposite layer has exfoliated clay platelets dispersed within a polymer matrix; and releasing or allowing to be released the active ingredient from the extended release polymeric dispenser.

"Glass transition temperature" or "$T_g$" as used herein, means the temperature at or above which a glassy polymer undergoes segmental motion of the polymer chain. Glass transition temperatures of a polymer are estimated by the Fox equation *[Bulletin of the American Physical Society* 1, 3 Page 123 (1956)], as follows:

$$\frac{1}{T_g} = \frac{w_1}{T_{g(1)}} + \frac{w_2}{T_{g(2)}}$$

For a copolymer, $w_1$ and $w_2$ are the weight fraction of the two co-monomers, and $T_{g(1)}$ and $T_{g(2)}$ are the glass transition temperatures, in degrees Kelvin, of the two corresponding homopolymers. For polymers containing three or more monomers, additional terms ($w_n/T_{g(n)}$) are added. The $T_g$ of a polymer phase is calculated by using the appropriate values for the glass transition temperatures of homopolymers, such as those found, for example, in "Polymer Handbook", edited by J. Brandrup and E. H. Immergut, Interscience Publishers. The values of Tg reported herein are calculated based on the Fox equation.

The use of the term "(meth)" followed by another term such as acrylate refers to both acrylates and methacrylates. For example, as used herein, the term "(meth)acrylate" refers to either acrylate or methacrylate, the term "(meth)acrylic" refers to either acrylic or methacrylic, and the term "(meth)acrylamide" refers to either acrylamide or methacrylamide.

Clays are commonly provided as particles having compositions based on hydrated aluminum silicates. The dimensions of the clay particles are typically in the range from 100 nanometers (nm) to 10 microns. Certain clay particles have structures containing multiple layers or stacks of clay platelets. Under suitable conditions, the stacks of clay platelets can be partially or completely separated to individual clay platelets. As used herein, the term "exfoliated clay" refers to a clay in the form of separated clay platelets having only one dimension in the range of nanometers and the other two dimensions in a larger size range, such as 100 nanometers and greater. As used herein, the term "to exfoliate" refers to the process of separating individual platelets from a clay particle or a stack of clay platelets, wherein the clay platelets have only one dimension in the range of nanometers and the other two dimensions in a large size range, such as 100 nanometers and greater. Typical size ranges for the exfoliated clays are platelets having one dimension, referred to as the thickness, in the range of from 1 nm to 20 nm, preferably in the range of from 1.5 nm to 15 nm, and more preferably in the range of from 2 nm to 12 nm. The other two dimensions of the platelet, which form the two faces of the platelet, are larger than the platelet thickness, and are typically in the range of from 50 nm to 20 microns, preferably in the range of from 75 nm to 15 microns, and more preferably in the range of from 100 nm to 10 microns. Surface areas for a platelet face are typically in the range of 2500 square nanometers to 400 square microns.

Nanocomposites are compositions containing a dispersed material that has one or more dimensions, such as length, width, or thickness, in the nanometer size range. Polymer-clay nanocomposites typically are characterized as being one of several general types: an intercalated nanocomposite, an exfoliated nanocomposite, or combinations thereof. The term "intercalated nanocomposite" as used herein, describes a nanocomposite that is characterized by the regular insertion of the polymer in between the clay layers, wherein the individual clay platelets are not completely separated from the clay particle. The term "exfoliated nanocomposite", as used herein, describes a nanocomposite wherein the clay is dispersed in a polymer matrix mostly as individual platelets having a single dimension, the thickness, in the nanometer size range. The exfoliated nanocomposite maximizes the polymer-clay interactions as the entire surface of the clay platelet is in contact with the polymer matrix. This modification often leads to the most dramatic changes in the mechanical and physical properties of the resultant polymer. In contrast, the term "conventional composite", as used herein, describes a composite in which the clay acts as a conventional filler and is not dispersed on a nanometer size scale. Generally, the exfoliated nanocomposites provide improvements of greater magnitude in mechanical or physicl properties than conventional composite materials. In certain embodiments of the present invention, some portion of the clay in the polymer-clay nanocomposite optionally exists as structures larger than exfoliated or intercalated composites.

The extended release dosage form and the extended release polymeric dispenser of this invention contain a polymer-clay nanocomposite having exfoliated clay platelets dispersed in a polymer matrix. The polymer matrix is a continuous solid phase in which the exfoliated clay platelets form a discontinuous phase within. In the extended release dosage form, the active ingredient is contained in the polymer-clay nanocomposite. The extended release dosage form is characterized as having a slower release rate for the active ingredient than a conventional dosage form containing the polymer matrix and the active ingredient but not containing the exfoliated clay dispersed in the polymer matrix. In the extended release polymeric dispenser, the polymer-clay nanocomposition partially or completely encapsulates a reservoir of the active ingredient. The extended release polymeric dispenser is characterized as having a slower release rate for the active ingredient than a conventional polymeric dispenser, which has a polymer matrix that is absent exfoliated clay, encapsulating the reservoir of the active ingredient.

Although not wishing to be limited to a particular theory, the inventors believe that one mechanism for providing the extended release of the active ingredient from the extended release dosage form or the extended release polymeric dispenser is slower diffusion of the active ingredient either through or from the polymer-clay nanocomposition compared to diffusion from a polymer matrix not containing the exfoliated clay. The platelets of the exfoliated clay dispersed the polymer matrix are believed to provide barriers to the movement of the active ingredient in the polymer matrix, thus increasing the diffusion pathlength or the tortuosity of the active ingredient through the polymer-clay nanocomposite, and leading to a slower rate of diffusion of the active ingredient from the polymer-clay nanocomposite. In comparison to nonexfoliated particles, the exfoliated clay platelets are particularly effective for reducing the diffusion of the active ingredient as they have large surface areas in relation to their volume or weight. Low levels of the exfoliated clay in the polymer matrix, either on a weight or a volume basis, provide a large number of barriers to the diffusive movement of the active ingredient in the polymer matrix, and lead to decreased release rates of the active ingredient from the extended release dosage form or extended release polymeric dispenser. Alternatively, the inventors believe that in select extended release dosage forms of this invention or select extended release polymeric dispensers of this invention, alternative mechanisms for providing extended release of the active ingredient are decreased rates of dissolution or swelling of the polymer-clay nanocomposite. In these select compositions or select articles, the polymer-clay nanocomposite swells or dissolves in the presence of a solvent or water, resulting in the release of the active ingredient from the polymer-clay nanocomposite, or from a reservoir encapsulated by the polymer-clay nanocomposite. The presence of the exfoliated clay platelets within the polymer matrix is believed to inhibit the movement of the solvent or water into the polymer-clay nanocomposite, resulting in slower rates of swelling or dissolution of the polymer-clay nanocomposite compared to a polymer matrix that does not contain exfoliated clay platelets. Alternatively, extended release of the active ingredient from the extended release dosage form or the extended release polymeric dispenser is provided by two or more different mechanisms, such as a combination of diffusion of the active ingredient from the polymer-clay nanocomposite and swelling of the polymer-clay nanocomposite by water; or a combination of diffusion of the active ingredient from the polymer-clay nanocomposite and dissolution of the polymer-clay nanocomposite.

The polymer matrix of the polymer-clay nanocomposite is any polymer that can be prepared containing exfoliated clay particles and providing extended release of the active ingredient from the polymer-clay nanocomposite. Various types of polymers are suitable for the polymer matrix including addition polymers and condensation polymers. Suitable polymers include homopolymers, copolymers, branched polymers, crosslinked polymers, interpenetrating polymer networks, block copolymers, graft copolymers, and blends of two or more polymers. The polymer matrix is optionally prepared from two or more different polymers. Processes to prepare the polymer matrix include, but are not limited to, bulk polymerization, solution polymerization, interfacial polymerization, emulsion polymerization, and suspension polymerization. Continuous, semicontinuous, and batch polymerization processes are contemplated.

Addition polymers are typically prepared by the polymerization of suitable ethylenically unsaturated monomers. Suitable addition polymers for the polymer matrix include polymers prepared from ethylenically unsaturated monomers such as linear, branched, and cyclic alkyl esters of (meth)acrylic acid; hydroxalkyl esters of (meth)acrylic acid; alkoxyalkyl (meth)acrylate; (meth)acrylonitrile; (meth)acrylamide; styrene; alpha-methyl styrene; vinyl esters such as vinyl acetate and vinyl versatate; alkenes such as ethylene, propylene, butene; dienes such as butadiene; ethylenically unsaturated acid containing monomers such as (meth)acrylic acid, itaconic acid, fumaric acid, phosphoethyl (meth)acrylate, vinyl sulfonic acid, and salts thereof; ethylenically unsaturated amine containing monomers such as dimethyl aminoethyl (meth)acrylate, t-butyl aminoethyl (meth)acrylate, and salts thereof; multiethylenically unsaturated monomers such as allyl (meth)acrylate, tripropylene glycol di(meth)acrylate, 1,6-hexanediol di(meth)acrylate, divinylbenzene, and trimethylolpropane tri(meth)acrylate; vinyl chloride; vinylidene chloride; ethylenically unsaturated anhydride monomers such itaconic anhydride; and mixtures thereof. Examples of suitable addition polymers include (meth)acrylic (co)polymers, vinyl acetate polymer, vinyl/acrylic copolymers, styrene/acrylic copolymers, ethylene/vinyl acetate copolymers, styrene/butadiene copolymers, polyvinyl alcohol, polyethylene, polypropylene, polytetrafluoroethylene, and polyvinyl chloride. Other suitable addition polymers include polyalkylene oxides such as, for example, poly(ethylene oxide), and poly(propylene oxide).

Suitable condensation polymers include polyesters, polyurethanes, polyureas, polyethers, polyamides, polysiloxanes, cellulose, polylactates, polycarbonates, phenol-formaldehyde resins, polyacetals, polyanhydrides, silicones, polysulfides, polyphosphates, and polyphosphate esters. Suitable silicone polymers include crosslinked silicone polymers.

The choice of polymer to prepare the polymer matrix is dependent upon one or more factors, such as the intended end use of the extended release dosage form or the extended release polymeric dispenser, the ability to incorporate the active ingredient into the polymer-clay nanocomposite, the diffusion rate of the active ingredient in the polymer-clay nanocomposite, and the extent of swelling or dissolution of the polymer matrix by a solvent or water. Physical properties that may affect the choice of polymer include the glass transition temperature or the molecular weight of the polymer. Polymers having low glass transition temperatures, such as in the range of -80°C to -10 °C are suitable for implants, especially when crosslinked. Hard polymers, such as those having glass transition temperatures in the range of from 30 °C to 140 °C are useful in the preparation of ion exchange resins for extended release or erodable polymer matrices. The weight average molecular weight of the polymer may be in the range of from 1,000 to greater than 2,000,000. Suitable polymers for erodable polymer matrices include polymers having weight average molecular weights in the range of from 1,000 to 100,000. The polymer may be crosslinked to modify the relase rate or to increase durability. Further, the polymer may be modified by post functionalization to include functional groups that provide specific properties to the polymer, such as adhesion, hydrophilicity, or binding groups for the active ingredient.

Suitable clays for preparing the polymer-clay nanocomposite include any natural or synthetic layered mineral capable of being exfoliated. Examples of such clays include, for example, layered silicate minerals such as smectite, phyllosilicate, montmorillonite, saponite, beidellite, montronite, hectorite, stevensite, vermiculite, kaolinite, and hallosite. A preferred clay is montmorillonite. Some non-limiting examples of synthetic minerals, or synthetic phyllosilicates, include LAPONITE™ clay, which is manufactured by Laporte Industries, LTD. of Charlotte, North Carolina, madadite, and fluorohectorite. Suitable clays are commonly treated with a surface treatment, such as quaternary ammonium surfactant. Suitable levels of exfoliated clay platelets in the polymer-clay nanocomposite are in the range of from 1 to 40 weight %, preferably in the range of from 3 to 35 weight %, and most preferably in the range of from 5 to 30 weight %, based on the weight of the polymer-clay nanocomposite. One useful range is 10 to 40 weight % of the exfoliated clay platelets, based on the weight of the polymer-clay nanocomposite. Suitable levels of exfoliated clay platelets in the polymer-clay nanocomposite layer of the extended release polymeric dispenser are in the range of from 1 to 40 weight %, preferably in the range of from 3 to 35 weight %, and most preferably in the range of from 5 to 30 weight %, based on the weight of the polymer-clay nanocomposite layer. One useful range is 10 to 40 weight % of the exfoliated clay platelets in the extended release polymeric dispenser, based on the weight of the polymer-clay nanocomposite layer.

The active ingredient contained in the extended release dosage form or the extended release polymeric dispenser includes materials that are biologically active, chemically active, physically active, or combinations thereof. Biologically active materials include, but are not limited to, pharmaceutical compounds such as drugs, buffers, and vitamins; agricultural compounds such as insecticides, pesticides, herbicides, and mildewcides; and biocides such as marine antifoulants. Chemically active materials include, but are not limited to, catalysts; initiators; inhibitors; stabilizers; and pH adjusting agents. Physically active materials include, but are not limited to, scents such as perfumes; and organoleptic substances such as flavors; and dyes.

Examples of pharmaceutical compounds useful as the active ingredient in the extended release dosage form or the extended release polymeric dispenser include antimicrobial agents such as ciprofloxacin, perfloxacin, ofloxacin, norfloxacin, phenoxymethyl penicillin, ampicillin, amoxycillin, erythromycin, cloxacillin, roxithromycin, azithromycin, cephalexin, cefadroxil, cerfuoxime, cerfuoxime axetil, cefixime, co-trimoxazole, acyclovir, cefaclor, clofazimin, fluconazole, griseofulvin, and ketoconazole; antiprotozoal agents such as metronidazole, tinidiazole, quinine, chloroquine, primaquine, sulfadoxine, and pyrimethanime; anthelmintic agents such as mebendazole; cardiovascular drugs such as amlodipine, diltiazem, atenolol, lisinopril, lovastatin, gemfibrozil, nifedipine, enalapril, and propanolol; drugs that act on the central nervous system L-dopa, buspirone, dextropropoxyphene, pentazocine, morphine derivatives, diazepam, lorazepam, alprazolam, haloperidol, chlorpromazine, and thioridazine; non-steroidal anti-inflammatory drugs such as diclofenac, ketorolac, piroxicam, ibuprofen, indomethacin, and naproxen; drugs used in treatment of respiratory disorders such as solbutamol, terbutaline, theophylline, and bromhexine; antihistaminics such as astemizole, terfenidine, and loratidine; prokinetic drugs such as metoclopramide, domperidone, and cisapride; corticosteroids such as prednisolone, dexamethasone, and betamethasone; steriod hormones such as stanazolol, and oral contraceptives; antiulcer drugs such as omeprazole, ranitidine, femotidine; central muscle relaxants such as carisoprodol, chlormezanone; anticancer drugs such as the alkylated agents merchlorthiamine, cyclophosphamide, ifosamide, chlorambucil, carmustine, hexamethylmelamine, thiotepa, busulfan, lomustine, semustine, streptozotocin, decarbazine; anticancer drugs such as the antimetabolites methatrexate, 5-flurouracil, floxuridine, cytosine arabinoside, 6-mercaptopurine, thioguanine, and pentostatin; anticancer drugs that are natural products such as vincristine, vinblastin, etoposide, dectinmycin, daunorubicin, doxorubicin, epirubicin, idarubicin, bleomycin, methramicin, mitomycin, and L-asparaginase; other anticancer drugs such as cisplatin, carboplatin, mitoxantrone, hydroxyurea, procarbazine, mitotane, and aminoglutathimide; and hormones and hormone antagonists such as prednisilone, hydroxyprogestirone, medroxyprogestirone, megestrol, diethyl stilbestrole, ethinyl estradiol, tamoxifen, testosterone, fluoxymestrone, flutamide, and leuprolide.

Examples of vitamins useful as the active ingredient in the extended release dosage form or the extended release polymeric dispenser include vitamin A, vitamin C, vitamin E, and vitamin K.

In the extended release dosage form, the active ingredient is contained within the polymer-clay nanocomposite. The active ingredient may be soluble in the polymer matrix, dispersed as multiple domains within the polymer matrix, absorbed onto the surfaces of the exfoliated clay platelets, or combinations thereof.

The extended release polymeric dispenser includes a reservoir of the active ingredient that is encapsulated by the polymer-clay nanocomposite layer. Optionally, the extended release polymeric dispenser also includes active material in the polymer-clay nanocomposite layer encapsulating the active ingredient reservoir.

In one embodiment, the extended release polymeric dispenser has a polymer-clay nanocomposite layer that completely encapsulates the active ingredient reservoir. Release of the active ingredient from the reservoir may be by transport of the active ingredient through the polymer-clay nanocomposite layer, dissolution or swelling of the polymer-clay nanocomposite layer, or a combination thereof.

In another embodiment, the extended release polymeric dispenser has a polymer-clay nanocomposite layer that partially encapsulates the active ingredient reservoir. In this embodiment, the active ingredient may be released by one or more of the following processes: from the section of the reservoir area that is not encapsulated by the polymer-clay nanocomposite layer, by transport of the active ingredient through the polymer-clay nanocomposite layer, or dissolution or swelling of the polymer-clay nanocomposite layer.

The polymer-clay nanocomposite may be prepared by various mixing or polymerization processes. Mixing processes include the dispersion and exfoliation of clay particles into the polymer matrix, such as melt blending and extrusion processes. Shear mixing is commonly employed to exfoliate the clay particles into clay platelets, in particular high shear mixing processes. Other suitable mixing processes include the use of moving media mills to exfoliate the clay particles into clay platelets. The moving media, such as sand, polymeric particles, ceramic balls, metal oxide beads, or metal shot, collide with the clay particles with the resulting impacts leading to breakup of the clay particles into individual clay platelets. Typically, this occurs in the presence of high shear conditions. Examples of moving media mills include, but are not limited to bead mills, sand mills, airjet mills, roller mills, attritor mills, vibratory mills, ball mills, and planetary mills.

In one mixing process suitable for preparing the polymer-clay nanocomposite, the clay particles are first dispersed into a suitable solvent, exfoliated into clay platelets, and then combined with a solution containing polymer in solvent. Removal of the solvent from the exfoliated clay-polymer solution results in the formation of the polymer-clay nanocomposite.

Polymerization processes suitable for preparing the polymer-clay nanocomposite include the dispersion and exfoliation of clay particles into polymer precursors such as monomer, followed by the polymerization of the polymer precursors to form the polymer-clay nanocomposite. The clay platelets may be formed prior to admixing with the polymer precursors or in the presence of the polymer precursors. Polymerization may be induced by the conventional chemical initiators, actinic radiation, or electron beam. For example, clay particles are admixed with at least one ethylenically unsaturated monomer and then polymerized by electron beam radiation. Optionally, an additive for treating the surfaces of the clay particles to aid in exfoliation is also added. The clay-monomer mixture is subjected to high shear mixing to disperse the clay particles and to exfoliate the clay particles into separate clay platelets. Next, the resulting exfoliated clay-monomer mixture is polymerized to provide the polymer-clay nanocomposite. Suitable polymerization processes include emulsion polymerization as disclosed in U.S. Patent Application 20020086908 A1.

X-ray diffraction techniques are useful for characterizing the structural regularity of stacks of clay platelets, and the structural irregularity of the exfoliated clay platelets in the polymer-clay composition. As the clay particles have stacks of uniformly spaced clay platelets, an X-ray diffraction pattern of a polymer matrix containing clay particles has a diffraction peak corresponding to the spacing of the clay platelets in the clay particles. In the curable clay composition, the X-ray diffraction peak is diminished or absent indicating a decrease in the order of the clay platelets and corresponding to the presence of exfoliated clay platelets. U.S. Patent No. 5,554,670 discusses the use of X-ray diffraction techniques to monitor the degree of exfoliation of the clay particles.

Ultrasonification may be used to exfoliate the clay particles and prepare the polymer-clay nanocomposite.

The extended release dosage form may be prepared by loading the active ingredient into the polymer-clay nanocomposite. In one process, the active ingredient is loaded into the polymer-clay nanocomposite by a concentration gradient. The concentration gradient is established by contacting the polymer-clay nanocomposite with the active ingredient or a solution containing the active ingredient, and allowing the active ingredient to enter the polymer-clay nanocomposite. Heating may be employed to increase the loading rate of the active ingredient. In another loading process, the polymer-clay nanocomposite is mixed with the active ingredient, for example, by melt blending or using high shear mixing. In still another loading process, the polymer-clay nanocomposition is swollen or solubilized in a suitable solvent, the active ingredient is combined with the swollen or solubilized polymer-clay nanocomposite, and the solvent is removed to provide the extended release dosage form.

Alternatively, the extended release dosage form may be provided by preparing the polymer-clay nanocomposite in the presence of the active ingredient. For example, the polymer matrix, clay particles, and the active ingredient are combined and subjected to high shear mixing to disperse and to exfoliate the clay particles to provide the polymer-clay nanocomposite having exfoliated clay platelets dispersed in a polymer matrix, and the active ingredient contained in the polymer-clay nanocomposite. The mixture of polymer matrix, the clay particles, and the active ingredient optionally contains one or more solvents, an additive for treating the surfaces of the clay particles to aid in exfoliation, or other processing aides. Alternatively, the extended release dosage form may be prepared by polymerizing polymer precursors, exfoliated clay platelets, and the active ingredient.

A combination of processes may be used to prepare the extended release dosage form.

The extended release polymeric dispenser may be prepared by a coating process such as a fluidized bed process. In this process, the active ingredient is provided as particles such as granules or pellets. The particles are coated with a solution containing the exfoliated clay and the polymer matrix, and then dried. The coating of the polymer-clay nanocomposite partially or completely encapsulates the particles of the active ingredient. The release rate may be controlled by the thickness of the polymer-clay nanocomposite coating and the size of the active ingredient particles. Alternatively, the active ingredient is applied onto a seed particle to form an active ingredient layer as the reservoir. Next, the polymer-clay nanocomposite layer is applied to partially or completely encapsulate the active ingredient layer. Suitable seed particles include polymer particles and inorganic particles. The extended release polymeric dispenser may contain two or more polymer-clay nanocomposite layers that completely or partially encapsulate the active ingredient reservoir. Coextrusion or laminating processes are alternative methods to prepare the extended release polymeric dispenser.

The extended release dosage form may be provided in various physical forms or shapes such as particles, pellets, and sheets. Alternatively, the extended release dosage form may be applied onto a substrate, for example, as a coating. Extrusion processes or casting from solution are suitable processes for applying the extended release dosage form onto a substrate. Suitable substrates include paper, plastics, nonwoven substrates, and woven substrates.

In one embodiment, the extended release dosage form is an ion exchange resin that contains exfoliated clay. As used herein, the term "ion exchange resin" means any water insoluble polymer that can act as an ion exchanger and includes cationic exchange resins and anionic exchange resins. Ion exchange resins are manufactured in different forms, including spherical and nonspherical particles with size in the range of from 10 nanometers (nm) to 2 millimeters (mm). The nonspherical particles are frequently manufactured by grinding of the spherical particles, and have particles sizes in the range of from 1 micron to 200 microns. Active ingredients that have acidic or basic ionizable groups may be loaded into the ion exchange resin containing exfoliated clay by an ion exchange process.

One aspect of the invention provides a method of releasing an active ingredient from the extended release dosage form, including the steps of: providing an extended release dosage form; and releasing or allowing to be released the active ingredient from the extended release dosage form. Optionally, this method includes the step of applying the extended release dosage form prior to or during the step of releasing or allowing to be released the active ingredient. The extended release dosage form may be applied directly to a substrate, object, or subject that is intended target of the active ingredient. Direct application includes parenteral application and nonparenteral application, such as oral ingestion, implantation, application to the eye, mouth, lungs, respiratory tract, gastrointestinal tract, nasal area, rectum, urinary tract, and vagina. Alternatively, the extended release dosage form may be applied indirectly to a secondary substrate, object, or subject in proximity to the substrate, object, or subject of the intended target of the active ingredient. In indirect application, the active ingredient is transported after release to the intended target, for example, the release of an insect repellant or an aroma from a container including the extended release dosage form.

In one embodiment, the extended release dosage form is applied directly to the object or surface to be treated, for example, by spraying a solution or a dispersion containing the polymer matrix, exfoliated clay, and the active ingredient onto the intended object; forming the extended release dosage form on the intended object or surface; and releasing or allowing to be released the active ingredient from the extended release dosage form. The method of this embodiment is useful in the application of agricultural chemicals directly onto the surfaces of leaves; or the application of active ingredients such as perfumes or pharmaceutical compounds onto skin.

A different aspect of the present invention provides a method of releasing an active ingredient from the extended release polymeric dispenser, including the steps of: providing an extended release polymeric dispenser; and releasing or allowing to be released the active ingredient from the extended release polymeric dispenser. Optionally, this method includes the step of applying the extended release polymeric dispenser form prior to or during the step of releasing or allowing to be released the active ingredient. The extended release polymeric dispenser may be applied directly onto a substrate, object, or subject that is intended target of the active ingredient; or applied indirectly, as disclosed herein above.

The following examples are presented to illustrate the composition and the process of the invention. These examples are intended to aid those skilled in the art in understanding the present invention. The present invention is, however, in no way limited thereby.

Example 1: Extended Release Dosage Form
Containing Insecticide as the Active Ingredient

A mixture of 80 parts by weight of linear low density polyethylene, 10 parts by weight of hydrophobically modified montmorillonite clay (Cloisite™ 10A clay), and 10 parts by weight insecticide are processed through a heated 2-screw extruder, and forced through a 3 mm x 30 mm slot die to form an extended release insecticide release product. The extended release insecticide release product has exfoliated clay platelets dispersed in the linear low density polyethylene. A comparative insecticide release product is made by extruding a mixture of 90 parts by weight of linear low density polyethylene and 10 parts by weight insecticide. The release of the insecticide is measured over a period of six months. The extended release insecticide product, which contains exfoliated clay dispersed in the linear low density polyethylene, releases the insecticide at a rate that meets or exceeds the minimum necessary use level of the insecticide for the full six month period. In contrast, the comparative insecticide release product, which does not contain exfoliated clay, initially releases the insecticide at a rate that meets or exceeds the minimum necessary use level of the insecticide. After three months, the release rate of the insecticide from the comparative insecticide release product decreases and is insufficient to maintain the minimum necessary use level for the remainder of the six month period.

Example 2: Extended Release Polymeric Dispenser
Containing Ibuprofen as the Active Ingredient

An aqueous suspension is prepared by admixing 2 parts by weight of Montmorillonite clay, 8 parts by weight hydroxyethylcellulose, and 90 parts by weight water. The aqueous suspension is milled in a stirred bead mill until the clay is exfoliated.
Solid ibuprofen is ground and sieved to an average particle diameter of approximately 1 mm. The particles are suspended in a fluidized bed dryer with air at a temperature of 75 °C. The aqueous suspension containing the exfoliated clay in hydroxyethylcellulose solution is sprayed onto the ibuprofen particles in the fluidized bed drier until a dried coating thickness of 0.5 to 1 mm is built up around the ibuprofen particles. The resulting coated ibuprofen particles are cooled to room temperature to provide the extended release polymeric dispenser. The extended release polymeric dispenser of this example has ibuprofen as the active ingredient and a layer of hydroxyethylcellulose containing exfoliated clay platelets as the polymer-clay nanocomposite layer that encapsulates the ibuprofen reservoir. The extended release polymeric dispensers of this example are packed into gelatin capsules to allow oral delivery of the ibuprofen.
A comparative polymeric dispenser is prepared by the same general procedure except that the ibuprofen particles are sprayed with a hydroxyethylcellulose solution that does not contain exfoliated clay. The comparative release polymeric dispenser has a dried coating thickness of 0.5 to 1 mm. The comparative polymeric dispensers are packed into gelatin capsules to allow oral delivery of the ibuprofen.
Ibuprofen is released from this capsule more slowly and evenly than in a similar capsule that does not include the exfoliated montmorillonite clay.

Example 3 - Preparation of Extended Release Dosage Form Containing Nicotine as the Active Ingredient

Ion exchange resin beads are prepared by the following suspension polymerization process. A monomer solution is prepared by admixing 509 g methacrylic acid and 41.8 g of 55 wt. % divinyl benzene. Next, 48.9 g of hydrophobically modified montmorillonite clay is slowly added to the monomer solution with sufficient stirring to prevent clumping and then subjected to high shear mixing to exfoliate the clay. Stirring is continued until the viscosity is approximately constant. Then, 1.7 g of t-butyl peroctoate, 1.7 g of t-amylperbenzoate, and 1.7 g of bis (4-t-butylcyclohexyl) peroxydicarbonate are added to the monomer solution. In a reaction vessel, the aqueous solution is prepared by dissolving 396 g of sodium chloride in 1100 g of water and then adding 6 g of sodium carboxymethylcellulose (NaCMC). The aqueous solution is agitated until the NaCMC is dissolved. The monomer solution is then added to the reaction vessel and the contents of the reaction vessel is agitated at 500 rotations per minute (rpm) for 45 minutes to create a dispersion of monomer droplets in the aqueous solution. The agitation rate is then reduced to 320 rpm. The contents of the reaction vessel is then heated according to the following temperature profile: increase the temperature from 20 °C to 40 °C at 1 °C/min, then increase the temperature to 104 °C at 0.1 °C/min. The resulting ion exchange resin beads are then transferred to a column and washed with deionized water upflow until the effluent is clear and the concentration of sodium chloride is <10 ppm. The washed ion exchange resin beads are then dried at 105 °C until the moisture content is <5 %w/w. The ion exchange resin beads are polymer-clay nanocomposites having 8.1 weight % exfoliated clay platelets dispersed in the polymer matrix. The ion exchange resin beads have a diameter in the range of from 200 to 900 microns.
Nicotine is loaded into the ion exchange resin beads by first preparing a nicotine solution containing 8 g of nicotine dissolved in 90 g water and 10 g ethanol. Next, 36 g of the ion exchange resin beads of Example 3 is added to the nicotine solution. The mixture is agitates at room temperature for 24 hours. The mixture is filtered and the ion exchange resin beads are washed with water to remove any residual nicotine from the outside of the beads. The ion exchange resin beads of Example 3 contain nicotine as the active ingredient.

A comparative dosage form is prepared by the general procedure for Example 3 except that hydrophobically modified montmorillonite clay is not included in the polymerization process, and is then load with nicotine. The comparative ion exchange resin beads are the polymer matrix and do not contain clay. The comparative ion exchange resin beads have a diameter in the range of from 200 to 900 microns. The comparative dosage form contains nicotine as the active ingredient.

The extended release dosage form of Example 3 and the comparative dosage release form are measured to determine the release rates and the durations of release of nicotine. To 1000 ml of a 0.9 wt. % saline solution at 37 °C, the extended release dosage form of Example 3 or the comparative dosage release form is added. Samples of the supernatant are removed at frequent intervals and analyzed for the amount of nicotine. A comparison of the release rate profiles show that the nicotine is released more slowly from those of Example 3 than the comparative dosage release form. Further, the extended release dosage form of Example 3 shows continued release for a longer period of time than the comparative dosage release form.

**Claims**

1. An extended release dosage form comprising:

   a) a polymer-clay nanocomposite having exfoliated clay platelets dispersed in a polymer matrix; and
   c) an active ingredient, which is contained within said polymer-clay nanocomposite.

2. The extended release dosage form according to claim 1 wherein said polymer-clay nanocomposite comprises from 10 to 40 weight % of said exfoliated clay platelets, based on weight of said polymer-clay nanocomposite.

3. A method of releasing an active ingredient, comprising the steps of:

   a) providing an extended release dosage form comprising:

   i) a polymer-clay nanocomposite having exfoliated clay platelets dispersed in a polymer matrix and
   ii) said active ingredient, which is contained within said polymer-clay nanocomposite; and

   b) releasing or allowing to be released said active ingredient from said extended release dosage form.

4. The method according to claim 3 wherein said polymer-clay nanocomposite comprises from 10 to 40 weight % of said exfoliated clay platelets, based on weight of said polymer-clay nanocomposite.

5. An extended release polymeric dispenser comprising: a polymer-clay nanocomposite layer encapsulating a reservoir of an active ingredient; wherein said polymer-clay nanocomposite layer has exfoliated clay platelets dispersed within a polymer matrix.

6. The extended release polymeric dispenser according to claim 5 wherein said polymer-clay nanocomposite layer comprises from 10 to 40 weight % of said exfoliated clay platelets, based on weight of said polymer-clay nanocomposite layer.

7. A method of releasing an active ingredient, comprising the steps of:

   a) providing an extended release polymeric dispenser comprising a polymer-clay nanocomposite layer encapsulating a reservoir of said active ingredient, wherein said polymer-clay nanocomposite layer has exfoliated clay platelets dispersed within a polymer matrix; and
   b) releasing or allowing to be released said active ingredient from said extended release polymeric dispenser.

8. The method according to claim 7 wherein said polymer-clay nanocomposite layer comprises from 10 to 40 weight % of said exfoliated clay platelets, based on weight of said polymer-clay nanocomposite layer.

**European Patent Office**

## PARTIAL EUROPEAN SEARCH REPORT

**Application Number**

which under Rule 45 of the European Patent Convention EP 04 25 2228
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GERSTL Z ET AL: "Controlled release of pesticides into water from clay -polymer formulations" JOURNAL OF AGRICULTURAL AND FOOD CHEMISTRY, AMERICAN CHEMICAL SOCIETY. WASHINGTON, US, vol. 46, September 1998 (1998-09), pages 3803-3809, XP002205376 ISSN: 0021-8561 * page 3805, column 1, last paragraph - page 3806, column 1, paragraph 2 * ----- | 1-8 | A61K47/02 A61K9/00 |
| P,X | WO 03/039251 A (RUSKIN RODNEY) 15 May 2003 (2003-05-15) * page 5, line 25 - page 6, line 12 * * page 7, lines 7-26 * * page 9, lines 24-31; table 1 * ----- -/-- | 1-4 | |
| | | | **TECHNICAL FIELDS SEARCHED (Int.Cl.7)** A61K |

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 25 August 2004 | Vermeulen, S |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C07)

**European Patent Office**

**INCOMPLETE SEARCH SHEET C**

Application Number

EP 04 25 2228

Although the definition of claims 3, 4, 7 and 8 includes methods of treatment of the human/animal body (Article 52(4) EPC), the search has been carried out and based on the alleged effects of the compound/composition.

-----

Claim(s) searched incompletely:
       3,4,7,8

Claim(s) not searched:
       -

Reason for the limitation of the search (non-patentable invention(s)):

Article 52 (4) EPC - Method for treatment of the human or animal body by therapy

# EP 1 470 823 A1

**European Patent Office**

**PARTIAL EUROPEAN SEARCH REPORT**

**Application Number**

EP 04 25 2228

| | DOCUMENTS CONSIDERED TO BE RELEVANT | | | CLASSIFICATION OF THE APPLICATION (Int.Cl.7) |
|---|---|---|---|---|
| Category | Citation of document with indication, where appropriate, of relevant passages | Relevant to claim | | |
| A | US 6 461 423 B1 (BEALL GARY W ET AL) 8 October 2002 (2002-10-08) * column 4, lines 15-33 * * column 7, lines 11-28 * * column 19, lines 30-51 * * column 23, lines 28-34 * * column 24, lines 1-36 * * column 26, lines 41-44,65-67 * ----- | 1-8 | | |
| | | | | **TECHNICAL FIELDS SEARCHED** (Int.Cl.7) |

EPO FORM 1503 03.82 (P04C10)

12

**EP 1 470 823 A1**

## ANNEX TO THE EUROPEAN SEARCH REPORT
## ON EUROPEAN PATENT APPLICATION NO.

EP 04 25 2228

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2004

| Patent document cited in search report | | Publication date | Patent family member(s) | | Publication date |
|---|---|---|---|---|---|
| WO 03039251 | A | 15-05-2003 | EP | 1441588 A1 | 04-08-2004 |
| | | | WO | 03039251 A1 | 15-05-2003 |
| | | | US | 2003092817 A1 | 15-05-2003 |
| US 6461423 | B1 | 08-10-2002 | US | 6126734 A | 03-10-2000 |
| | | | US | 5830528 A | 03-11-1998 |
| | | | AT | 214683 T | 15-04-2002 |
| | | | CA | 2182557 A1 | 23-06-1997 |
| | | | DE | 69619946 D1 | 25-04-2002 |
| | | | DE | 69619946 T2 | 29-08-2002 |
| | | | EP | 0780340 A1 | 25-06-1997 |
| | | | JP | 9175816 A | 08-07-1997 |
| | | | US | 6083559 A | 04-07-2000 |

EPO FORM P0459

For more details about this annex : see Official Journal of the European Patent Office, No. 12/82

13